# EUROPÄISCHE PATENTSCHRIFT

(11) **EP 4 416 931 B1**
(45) Veröffentlichungstag und Bekanntmachung des Hinweises auf die Patenterteilung: **22.07.2026**
(21) Anmeldenummer: 22801777.8
(22) Anmeldetag: 13.10.2022
(51) Int. Cl.: H04N 23/68, H04N 7/18, A61B 90/20, G02B 21/36, G02B 27/64, G06T 7/246, G02B 21/00, A61B 34/20

(54) **MEDIZINISCHES VISUALISIERUNGSYSTEM UND VERFAHREN ZUR VIDEO-STABILISIERUNG BEI EINEM SOLCHEN SYSTEM**
MEDICAL VISUALIZATION SYSTEM AND METHOD FOR VIDEO STABILIZATION IN SUCH A SYSTEM
SYSTÈME DE VISUALISATION MÉDICALE ET PROCÉDÉ DE STABILISATION VIDÉO DANS UN TEL SYSTÈME

(30) Priorität: 14.10.2021 DE 102021126658
(43) Veröffentlichungstag der Anmeldung: 21.08.2024
(73) Patentinhaber: Carl Zeiss Meditec AG, 07745 Jena (DE)
(72) Erfinder: GEISSLER, Enrico, 07749 Jena (DE); BRENNER, Philipp, 76185 Karlsruhe (DE); SCHERER, Dominik, 73430 Aalen (DE); HILLENBRAND, Matthias, 07745 Jena (DE); STEFFEN, Joachim, 73463 Westhausen (DE); WOLF, Christian, 73431 Aalen (DE); KROLLA, Bernd, 67655 Kaiserslautern (DE)
(74) Vertreter: Patentanwälte Geyer, Fehners & Partner mbB
(86) Internationale Anmeldenummer: PCT/EP2022/078492
(87) Internationale Veröffentlichungsnummer: WO 2023/062121

(56) Entgegenhaltungen:
- EP-A1- 3 437 547
- JP-A- 2009 147 727
- JP-B2- 4 274 233
- US-A1- 2018 172 971
- US-A1- 2019 394 400

## Beschreibung

Die Erfindung bezieht sich auf ein medizinisches Visualisierungsystem, insbesondere ein OP-Mikroskopsystem, sowie ein Verfahren zur Video-Stabilisierung bei einem solchen System.

Bei medizinischen Visualisierungsystemen, z.B. in der Mikroskopie und insbesondere bei Operationsmikroskopen (OP-Mikroskopen), wird ein stabiles Live-Videobild auf einer Displayeinheit (beispielsweise einem Monitor, einer Mixed-Reality-Brille, einem digitalen Okular, einem Projektor, etc.) benötigt.

Es ist hierzu aus der EP 3 437 547 A1 bekannt, eine elektronische Bildstabilisierung durchzuführen, wobei als Bewegungserfassungseinrichtung entweder eine Bildauswertung oder ein Beschleunigungssensor eingesetzt wird, um Bewegungen des Operationsmikroskops und daraus erforderlichen Stabilisierungsbedarf zu erkennen.

Die US 2018/172971 A1 verwendet ebenfalls einen Beschleunigungssensor als Bewegungserfassungseinrichtung, um zu erkennen, ob das Bild stabilisiert werden muss. Sie führt dann eine mechanische Bildstabilisierung durch entsprechende Bewegung eines Optikkopfs des OP-Mikroskops aus. Es ist vorgesehen, verschiedene Bewegungen zu unterscheiden und insbesondere eine Schwingungserkennung anhand der Signale des Beschleunigungssensors durchzuführen. Anhand einer Frequenzanalyse werden verschiedene Schwingungsmuster unterschieden, beispielsweise Schwingungen, die von Gebäudeschwingungen herrühren, und Schwingungen durch Stöße gegen das OP-Mikroskop.

US 2019/394400 A1, die im Oberbegriff der unabhängigen Ansprüche berücksichtigt ist, betrifft ebenfalls die Bildstabilisierung und ordnet dazu einen Schwingungssensor als Bewegungserfassungseinrichtung im Operationsmikroskop an. Aus dessen Signalen wird die Art der Schwingung ermittelt und der Stabilisierungsbedarf festgestellt. Die Bildstabilisierung wird dann als elektronische Bildstabilisierung, d.h. durch geeignete Bearbeitung der Video-Bilddaten, oder als mechanische Bildstabilisierung, d.h. durch geeignete Verschiebung optischer Elemente oder eines Bildsensors, ausgeführt.

CN 1 13 132 612 A beschreibt ein Verfahren zur Bildstabilisierung, welches unterschiedliche Stabilisierungsmethoden für verschiedene Bildbereiche, dort Vorder- und Hintergrund, verwendet, um ein Verwackeln der Kamera mittels Bildbearbeitung auszugleichen. Dabei werden, zusätzlich zu Bildbewegungsdaten, auch Gyroskop-Daten der Kamera ausgewertet.

US 8 749 648 B1 offenbart unter anderem ein Verfahren, bei dem aus einem Bewegungssensor gewonnene Bewegungsdaten, die während der Aufnahme aufgezeichnet wurden, in einer nachgelagerten Bildverarbeitung verwendet werden, um das Video zu stabilisieren.

Die JP 2009 147727 A und JP 4 274233 B2 betreffen die Korrektur von Video-Daten unter Verwendung eines Verschiebungsvektors.

Bei der Operationsmikroskopie wird ein Live-Bild von einem Arzt benutzt. Zeitverzögerungen sind hierbei äußerst störend. Hier erweist sich der Stand der Technik als problematisch, da die Bildstabilisierung relativ rechenaufwendig ist und damit zu Zeitverzögerungen bei der Anzeige des Live-Videos führen kann. Zudem können im Stand der Technik Objektbewegungen schlecht von Mikroskopschwingungen unterschieden werden.

Der Erfindung liegt deshalb die Aufgabe zugrunde, eine verbesserte Bildstabilisierung für die Operationsmikroskopie bereitzustellen, welche die Probleme des Standes der Technik vermeidet.

Die Erfindung ist in den Ansprüchen 1 und 8 definiert. Sie stellt ein medizinisches Visualisierungsystem sowie ein Verfahren zur Video-Stabilisierung bei einem medizinischen Visualisierungsystem bereit. Das medizinische Visualisierungsystem hat einen Bildsensor. Weiter wird eine Bewegungserfassungseinrichtung eingesetzt, welche Bewegungen des Bildsensors erfasst und entsprechende Sensor-Bewegungsdaten erzeugt, während gleichzeitig ein Video-Bild eines Objektes mit dem medizinischen Visualisierungsystem erzeugt wird.

Soweit nachfolgend von einem OP-Mikroskop(system) die Rede ist, geschieht dies exemplarisch für ein medizinisches Visualisierungsystem.

Die Erfindung nutzt die Erkenntnis, dass zur Bildstabilisierung mehrere Bewegungen gleichzeitig auftreten können. Es kann eine Bewegung des Mikroskops relativ zum Objekt vorliegen.

Dies wäre beispielsweise eine Mikroskopschwingung. Es können aber auch Bewegungen des Objektes vorliegen, die entweder vollflächig oder teilweise im Bild auftreten. Ein Beispiel wären in der Operationsmikroskopie Blutgefäße, die mit der Herztätigkeit eine rhythmische Bewegung ausführen. Weiter kann es extern bewegte Elemente im Objekt geben, die üblicherweise im Vordergrund und damit möglicherweise auch unscharf auftreten. Zu denken ist im Falle der Operationsmikroskopie beispielsweise an Bewegung von chirurgischen Instrumenten oder Werkzeugen. Diese verschiedenen Komponenten bilden einen Bewegungsvektor und können durch eine Bildanalyse nicht voneinander unterschieden werden - dies gilt auch, wenn zusätzlich ein Beschleunigungssensor gemäß dem Stand der Technik verwendet werden soll, um den Bedarf für eine Bildstabilisierung zu erkennen.

Unter dem Begriff "Bewegungsvektor" wird hier ein Vektor verstanden, der sämtliche Bewegungen in den Video-Daten wiedergibt, unabhängig davon, ob sie durch die Bewegung des Mikroskops relativ zum Objekt, durch Bewegungen des Objektes selbst oder durch Bewegungen im Vordergrund des Bildes verursacht werden. Er lässt sich aus den Bild-Bewegungsdaten ermitteln. Die "Verschiebungsvektordaten" sind hingegen diejenigen Verschiebungsangaben, die nach der entsprechenden kombinierten Auswertung von Bild-Bewegungsdaten und Sensor-Bewegungsdaten erhalten wurden, und ausschließlich oder zu einem Anteil von mindestens 60 %, bevorzugt 70 %, ganz besonders bevorzugt 80 % und höchst bevorzugt 90 % durch Bewegungen im Objektfeld, also dem gewünschten abseparierten Anteil aus dem Bewegungsvektor, verursacht sind.

Die Korrektur kann sowohl als einfache Korrektur einer lateralen Verschiebung, als auch als komplexere Korrekturen, die unter dem Begriff Warp zusammengefasst werden können, ausgeführt werden. Die Verschiebungsvektoren bilden bevorzugt eine Matrix, welche eine komplexere Korrektur ermöglicht. Im einfachsten Fall wird der Mittelwert als laterale Verschiebung genutzt. In Verbindung mit der dritten Raumrichtung ist eine überlagerte Vergrößerungsänderung mittels Warp die Korrekturmaßnahme.

Die Erfindung kombiniert die Auswertung des Bildes und der Bewegungserfassungseinrichtung, um aus dem Bewegungsvektor diejenigen Anteile abzuseparieren, die durch eine Bewegung des Mikroskops relativ zum Objekt verursacht werden. Der Bewegungsvektor und damit die Bild-Bewegungsdaten zeigen Verschiebungen innerhalb der Video-Daten an, ist/sind also per se noch nicht hinsichtlich der interessierenden Mikroskopbewegung separiert. Durch die kombinierte Verwendung der Sensor-Bewegungsdaten und der Bild-Bewegungsdaten ist es möglich, den Bewegungsvektor anhand der Sensor-Bewegungsdaten zu gewichten und/oder filtern. Auf diese Weise werden die Verschiebungsvektordaten erzeugt, welche nunmehr eine Bewegung des Bildsensors wiedergeben, aber keine Bewegung im Objektfeld. Die Nutzung der Bewegungserfassungseinrichtung im kombinierten Ansatz ermöglicht damit eine Gewichtung bzw. ein Aussortieren von Bewegungsvektoren und damit die Abseparation des Anteils des Bewegungsvektors, der die Bewegung des Mikroskops selbst wiedergibt.

Durch die Kombination ist eine Genauigkeit erreicht, die über die Auflösung der Sensor-Bewegungsdaten hinausgeht.

Dieser Ansatz ist zudem nicht nur besonders rechensparend und mit wenig Zeitverzögerung verbunden, er hat darüber hinaus auch den weiteren Vorteil, dass eine große Vielfalt an Bewegungserfassungseinrichtungen verwendet werden kann, ohne dass die Auswertung anders ausgeführt werden müsste. Die Abseparierung des interessierenden Anteils ist gänzlich unabhängig von der Art der Bewegungserfassungseinrichtung. Damit erreicht man eine leichte Adaptierbarkeit bzw. Nachrüstungsmöglichkeit für bestehende OP-Mikroskope.

Die Bewegungserfassungseinrichtung kann einen oder mehrere der folgenden Sensoren/Techniken verwenden:
a. einen ein- bis sechsachsigen Beschleunigungssensor, der lineare Beschleunigungen entlang drei Achsen misst und sich auf der Bildaufnahmeeinheit oder dem Objekt befindet;
b. eine inertiale Messeinheit, welche lineare Beschleunigungen, z.B. entlang drei Achsen sowie Rotationen um drei Achsen misst ("Gyroskop") und ggf. noch das Magnetfeld misst (9DOF-absolute orientation sensors) und sich auf der Bildaufnahmeeinheit oder dem Objekt befindet;
c. eine Umfeldkamera mit weitem Blickwinkel, die ebenfalls auf der Bildaufnahmeeinheit sitzt, allerdings ein größeres oder anderes Bildfeld betrachtet (ggf. auch eine andere Richtung);
d. eine weitere Kamera oder allgemeiner ein externes Tracking-System, z.B. ein Laser-Tracking-System, welches nicht auf der Bildaufnahmeeinheit sitzt und die Bewegung der Bildaufnahmeeinheit von außen ermittelt. Hierfür können bei Bedarf auf der Bildaufnahmeeinheit zusätzlich Elemente wie Marker oder Retroreflektoren platziert werden;
e. eine Projektion von Markern/Muster ausgehend von der Bildaufnahmeeinheit und die Ermittlung der relativen Position der Marker/Muster zum Bildinhalt. Dabei können die Marker und der restliche Bildinhalt entweder im gleichen oder in einem bewusst trennbaren Spektralbereich (z.B. IR) liegen;
f. eine Projektion von Markern/Muster ausgehend von einem externen statischen Objekt;
g. ein Tracking von markanten Objektmerkmalen mit einem separatem Trackingsystem, wie beispielsweise ein Pupillentracking bei Operationsmikroskopen in der Ophthalmologie, und
h. einen zweiten Bildsensor, wie er ggf. in der Bildaufnahmeeinheit von Stereomikroskopsystemen vorhanden ist.

Bei OP-Mikroskopen ist der Bildsensor üblicherweise an einem Stativ oder Arm befestigt. Es ist dann bevorzugt, ein Schwingungsmodell des Stativs oder Arms zu verwenden, um die Verschiebungsvektordaten zu berechnen. Dieser Einsatz dient dazu, ein typisches Schwingungsverhalten des Bildsensors zugrunde zu legen, um aus diesem den Bewegungsvektor zu filtern/gewichten, um die Verschiebungsvektordaten zu ermitteln, die für die Korrektur der Video-Daten verwendet werden können. Das Schwingungsmodell dient nicht dazu, eine Analyse der Schwingungen auszuführen und insbesondere auf Schwingungen mit bestimmten Parametern zu prüfen.

In Ausführungsformen wird das Objekt mit einer vorgegebenen Gesamtvergrößerung abgebildet und auf einem Display angezeigt. Bevorzugt wird dabei ein das Verhältnis von optischer Vergrößerung (optischer Zoom) zu digitaler Vergrößerung (digitalem Zoom) ggf. neu eingestellt. Wurde keine Schwingung in der Ebene parallel zum Bildsensor um eine Ruhelage erkannt, so wird die vom Nutzer gewünschte Vergrößerung primär durch die optische Vergrößerung des Systems eingestellt. Hierdurch ergibt sich eine maximale Bildqualität. Wurde hingegen eine Schwingung parallel zum Bildsensor um eine Ruhelage erkannt, so kann teilweise optisch heraus- und digital hineingezoomt werden. Dadurch steht für den nachfolgenden Zyklus ein größerer Bereich auf dem Bildsensor zur Verfügung, der für nachfolgende Korrekturschritte verwendet werden kann und es ergibt sich eine optimierte Videostabilität trotz sich bewegender Bildaufnahmeeinheit.

Bei Schwingungen, die senkrecht zur Ebene des Bildfeldes, welches der Bildsensor erfasst, verlaufen, könnte eine Defokussierung auftreten. Ausführungsformen stellen deshalb bevorzugt eine Pupillenblende passend ein, denn die Pupillenblende beeinflusst bekanntermaßen die Schärfentiefe der Abbildung. Zieht man die Pupillenblende zu, erhöht sich die Schärfentiefe. Die Bildhelligkeit wird dann fachüblicher Weise durch Anpassen einer elektronischen Verstärkung konstant gehalten, d.h. beim Schließen der Pupillenblende wird die Bildverstärkung erhöht und umgekehrt. Zum Einstellen der Pupillenblende kann nun das Ergebnis der Schwingungsanalyse berücksichtigt werden. Die Blende des optischen Systems und die elektronische Verstärkung bzw. Belichtungszeit wird dann neu eingestellt. Wird keine Schwingung um eine Ruhelage senkrecht zum Bildfeld (entlang der optischen Achse) erkannt, so wird die Blende des Systems weit geöffnet, um eine bestmögliche Bildqualität zu erhalten. Wird hingegen eine Schwingung um eine Ruhelage senkrecht zum Bildfeld erkannt, so wird die Bildblende ggf. auf einen kleineren Wert eingestellt, um die Schärfentiefe zu erhöhen. Um eine ähnliche Bildhelligkeit zu erhalten, wird/werden ggf. die elektronische Verstärkung und/oder die Belichtungszeit adaptiert.

Ähnliches gilt für die Fokusebene des optischen Systems. Auch sie ist von Schwingungen senkrecht zur Ebene des Bildfeldes betroffen, von Schwingungen in der Ebene des Bildfeldes hingegen nicht. Die Fokusebene des optischen Systems wird neu eingestellt. Wird eine Schwingung um eine Ruhelage senkrecht zum Bildfeld (entlang der optischen Achse) erkannt, so wird die Fokusebene entsprechend eines für den nächsten Belichtungszeitraum vorhergesagter Position neu eingestellt und/oder die Schärfentiefe wird durch teilweises Schließen der Blende so eingestellt, dass ein hinreichend großer/der gesamte vertikale Schwingungsbereich hinreichend scharf abgebildet wird.

Das beschriebene OP-Mikroskopsystem bzw. das beschriebene Verfahren hat weiter den Vorteil, dass immer nur eine Schwingung des OP-Mikroskops erkannt wird. Eine Bewegung des Objektes wird nicht mehr fälschlich aus dem Bewegungsvektor in die Bewegungsvektordaten übernommen, wie dies beispielsweise bei einer reinen Bildanalyse der Fall sein kann. Weiter ist es nicht erforderlich, dass, wie in EP 3 437 547 A1, ein Bildsensor verwendet werden muss, der mehr Pixel besitzt als die Anzeigeeinrichtung, welche zur Darstellung des Videos verwendet wird. In Ausführungsformen haben Bildsensor und Display die gleiche Pixelzahl.

Das beschriebene Konzept erreicht weiter eine Bildstabilisierung in 3D, d.h. auch längs der optischen Achse. Eine Bildunschärfe, die von Schwingungen erzeugt wird, kann damit korrigiert werden.

Gemäß der Erfindung wird eine Filterung anhand der Sensor-Bewegungsdaten vorgenommen. Es wird ein Bewegungsvektor-Bereich definiert, und nur Bild-Bewegungsdaten, die innerhalb dieses Bereichs liegen, werden für die Verschiebungsvektordaten verwendet. Dabei ist in der Filterung nicht nur eine Ja/Nein-Auswahl, sondern auch eine Gewichtung der Bild-Bewegungsdaten möglich, z.B. eine Abstands-Gewichtung. Auch machine learning kann zum Einsatz kommen, um die Filterung zu verbessern.

Soweit die Erfindung hier unter Bezugnahme auf ein OP-Mikroskop beschrieben wird, muss das am OP-Mikroskop ausgeführte Verfahren nicht zwingend mit einem chirurgischen oder diagnostischen Verfahren verknüpft sein. In Ausführungsformen wird kein Therapie- oder Diagnoseverfahren am lebenden, menschlichen oder tierischen Körper ausgeführt. Beispiele für solche nicht-therapeutischen und nicht-diagnostischen Einsätze eines OP-Mikroskops finden sich insbesondere in der Ophthalmologie, z.B. bei der Betrachtung des Augenhintergrundes, oder bei der Vor-Abklärung eines späteren Operationsfeldes, z.B. in der Mundhöhle, im Nasen-Rachenraum oder im Bereich des Ohres. Gleichermaßen kann ein OP-Mikroskop auch zur Präparation eines Transplantats, d.h. am nicht-lebenden menschlichen Körper eingesetzt werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine Schemadarstellung eines OP-Mikroskopsystems,
- Fig. 2: ein Blockschaltbild für ein Verfahren zur Video-Stabilisierung,
- Fig. 3: eine Schemadarstellung zur Erläuterung einer Erzeugung von Verschiebungsvektordaten, und
- Fig. 4 und 5: Abwandlungen des Mikroskops der Fig. 1.

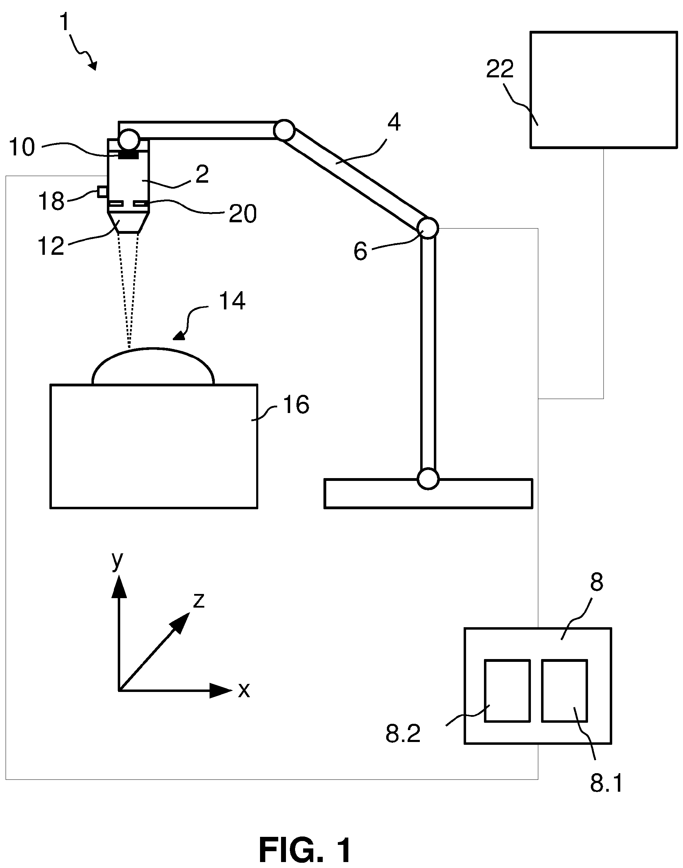

Fig. 1 zeigt schematisch ein OP-Mikroskop 1, das zusammen mit einem Beschleunigungssensor und einer Steuereinrichtung ein OP-Mikroskopsystem bildet. Das OP-Mikroskop 1 umfasst einen Mikroskopkopf 2, der an einem Arm 4 befestigt ist. Der Arm 4 ist über Gelenke 6 verstellbar, so dass die Lage des Mikroskopkopfs 2 im 3D-Raum eingestellt werden kann. Die Gelenke 6 verfügen dazu über Antriebe, um einzelne Segmente des Armes 4 gegeneinander verstellen zu können. In der Regel sind beim OP-Mikroskop 1 sechs Freiheitsgrade der Verstellung möglich, nämlich drei der Translation und drei der Rotation. Die Gelenke 6 sind hinsichtlich ihrer Antriebe mit einer Steuereinrichtung 8 verbunden, welche die Stellung des Arms 4 und damit des Mikroskopkopfs 2 einstellt. Der Mikroskopkopf 2 ist ebenfalls mit der Steuereinrichtung 8 verbunden, die einen Prozessor 8.1 sowie einen Speicher 8.2 aufweist. Sie steuert den Betrieb des OP-Mikroskops 1 und führt, wie nachfolgend noch erläutert werden wird, insbesondere eine Bildstabilisierung aus.

Der Mikroskopkopf 2 umfasst einen Bildsensor 10, auf dem mittels eines Objektivs 12, das i.d.R. als Zoom-Objektiv ausgeführt ist, ein Objekt 14, z.B. ein Teil eines Patienten, abgebildet wird, welches sich auf einem Tisch 16 befindet, üblicherweise einem OP-Tisch.

Im Mikroskopkopf 2 ist zur Einstellung eine Blende 20 vorgesehen, die als Pupillenblende ausgeführt ist und die Lichtmenge einstellt, welche durch das Objektiv 12 auf den Bildsensor 10 fällt. Üblicherweise weist das OP-Mikroskop 1 auch noch weitere Elemente auf, beispielsweise eine Beleuchtungsquelle etc. Dies ist in der Schemadarstellung der Fig. 1 nicht eingezeichnet, da für die hierzu beschreibenden Details nicht weiter relevant.

Der Mikroskopkopf 2 ist über eine nicht weiter bezeichnete Steuerleitung mit der Steuereinrichtung 8 verbunden, welche den Betrieb des OP-Mikroskops 1 am Mikroskopkopf 2 steuert, insbesondere die Aufnahme von Bilddaten durch den Bildsensor 10 und die Stellung des Objektivs 12 sowie der Pupillenblende 20. Die Steuereinrichtung 8 liest weiter den Beschleunigungssensor 18 aus, der mit dem Bildsensor 10 starr verbunden ist, so dass er die Beschleunigungen misst, welche am Bildsensor 10 auftreten.

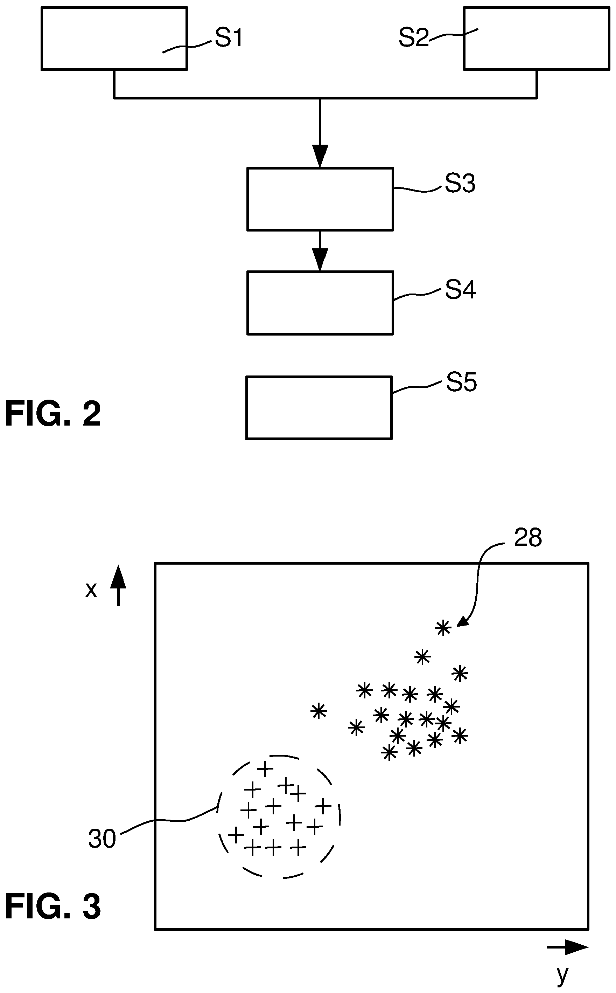

Mit Hilfe dieses OP-Mikroskopsystems werden beim Betrieb des OP-Mikroskops 1 Video-Daten aufgenommen und von der Steuereinrichtung 8 verarbeitet und dann auf einem Display 22 angezeigt. Hierbei wird eine Video-Stabilisierung gemäß dem in Fig. 2 schematisch dargestellten Verfahren ausgeführt. In einem Schritt S1 wird die Bildinformation durch den Bildsensor 10 aufgenommen, d.h. es werden die Video-Daten erfasst, welche das Objekt 14 in einer einstellbaren Vergrößerung zeigen. Aus den Video-Daten werden Bild-Bewegungsdaten durch bekannte Bildauswertung ermittelt. Sie zeigen Bewegungen im Bild an.

Gleichzeitig dazu wird in einem Schritt S2 eine Sensor-Bewegungsinformation gewonnen, indem der Beschleunigungssensor 18 ausgelesen wird. Im Schritt S2 wird die Position des Bildsensors 10 ermittelt und die sich daraus ergebenden Sensor-Bewegungsdaten berechnet.

Im Schritt S3 wird anhand der Kombination von Bild- und Sensor-Bewegungsdaten ein möglichst exakter Verschiebungsvektor ermittelt. Dabei werden nicht nur die reinen Bilddaten aus Schritt S1 verwendet (wie in EP 3 437 547 A1). Auch wird die Auswertung der Bilddaten nicht lediglich von einer vorherigen Klassifikation der Sensor-Bewegungsdaten abhängig gemacht, wie es im Stand der Technik bekannt wäre. Vielmehr wird zuerst anhand der Bild-Bewegungsdaten ein Bewegungsvektor berechnet, der dann anhand der Sensor-Bewegungsdaten gewichtet und/oder gefiltert wird. Insbesondere wird in einem Schritt S4 anhand der Sensor-Bewegungsdaten ein Bewegungsvektor-Bereich gefiltert, in dem Bild-Bewegungsdaten liegen können, die wahrscheinlich von einer Bewegung des Mikroskops herrühren. Außerhalb dieses Bereichs liegende Bild-Bewegungsdaten werden unterdrückt und tragen nicht zu den Verschiebungsvektordaten bei.

In diesem Zusammenhang sei darauf hingewiesen, dass die Bild- und Sensor-Bewegungsdaten jeweils in der Regel als Bewegungsvektoren bzw. -vektorgruppe (für verschiedene Bildpunkte oder Teilobjekte) aufgefasst werden können. Die kombinierte Zusammenfassung dieser Daten erlaubt eine Gewichtung und die gewünschte Unterscheidung von Bewegungsvektoren, die nicht von der Mikroskopbewegung herrühren. Es ist damit z. B. vermieden, dass eine globale Bewegung des beobachtenden Objekts als Bewegung der Bildaufnahmeeinheit interpretiert werden kann.

Als weitere positive Eigenschaft der Einbindung einer zweiten Information (aus einem Sensor oder einer Systeminformation) ergibt sich eine Reduzierung des notwendigen Rechenaufwandes und damit eine Reduktion der Latenz der Videoübertragung.

Im Schritt S4 erfolgt z. B. eine algorithmische Bestimmung, ob eine Schwingung um eine Ruhelage mit zu korrigierender Amplitude vorhanden ist. Die Entscheidung eines solchen Algorithmus kann beispielsweise auf folgende Informationen zurückgreifen:
a. der ermittelte Verschiebungsvektor und ggf. weitere Sensordaten werden anhand der Sensor-Bewegungsdaten mit festgelegten Schwellwerten verglichen;
b. der Verlauf der Verschiebungsvektoren und ggf. weitere Sensordaten werden aus dem Speicherelement werden mit einem analytischen Modell (z.B. einer exponentiell abfallenden Schwingung) verglichen; und
c. der Verlauf der Verschiebungsvektoren und ggf. weitere Sensordaten werden aus dem Speicherelement werden auf bestimmte Muster untersucht, die über Methoden des maschinellen Lernens eine Aussage über das Vorhandensein einer Schwingung erlauben.

Fig. 3 zeigt schematisch eine Auswertung der Bewegungsvektoren auf Basis der Sensor-Bewegungsdaten. Es sind Bewegungswinkel in x- und y-Achse aufgetragen und die einzelnen Messpunkte sind Bewegungsvektoren 28, die sich aus den Bild-Bewegungsdaten ergeben. Nur die innerhalb des Bereiches 30 liegenden Bewegungsvektoren 28 (mit einem "+" eingetragen) werden zur Ermittlung der Verschiebungsvektordaten herangezogen. Die x- und y-Achsen sind damit die Winkelprojektionen in der zweidimensionalen Objekteebene. Die Unterscheidung der Bewegungsvektoren "+" und "*" erfolgt aufgrund der Sensor-Bewegungsdaten.

Natürlich ist dieses Vorgehen nicht auf eine zweidimensionale Analyse beschränkt, sondern kann auch die dritte Dimension, d. h. die Tiefendimension eines Objektfeldes berücksichtigen. Insbesondere kann eine Vektorlänge als Maß einer Güte der einzelnen Bewegungsvektoren mit einbezogen werden, um die final zu ermittelnden Verschiebungsvektordaten (z. B. durch Mittelwertbildung) mit möglichst hoher Präzision zu erhalten.

In einem optionalen nachgelagerten Schritt S5 wird das optische System des Mikroskopkopfs 2 optimiert:
a. das Verhältnis von optischer Vergrößerung (optischer Zoom 12) zu digitaler Vergrößerung (digitalem Zoom) wird ggf. neu eingestellt. Wurde keine Schwingung in der Ebene parallel zum Bildsensor 10 um eine Ruhelage erkannt, so wird die vom Nutzer gewünschte Vergrößerung primär durch die optische Vergrößerung des Systems eingestellt. Hierdurch ergibt sich eine maximale Bildqualität. Wurde hingegen eine Schwingung parallel zum Bildsensor 10 um eine Ruhelage erkannt, so kann teilweise optisch heraus- und digital hineingezoomt werden. Dadurch steht für den nachfolgenden Zyklus ein größerer Bereich auf dem Bildsensor 10 zur Verfügung, der für nachfolgende Korrekturschritte verwendet werden kann und es ergibt sich eine optimierte Videostabilität trotz sich bewegender Bildaufnahmeeinheit;
b. die Blende 20 des optischen Systems und die elektronische Verstärkung bzw. Belichtungszeit wird dann neu eingestellt. Wird keine Schwingung um eine Ruhelage senkrecht zum Bildsensor 10 (entlang der optischen Achse) erkannt, so wird die Blende 20 des Systems weit geöffnet, um eine bestmögliche Bildqualität zu erhalten. Wird hingegen eine Schwingung um eine Ruhelage senkrecht zum Bildsensor 10 erkannt, so wird die Blende 20 ggf. auf einen kleineren Wert eingestellt, um die Schärfentiefe zu erhöhen. Um eine ähnliche Bildhelligkeit zu erhalten, wird ggf. die elektronische Verstärkung und/oder die Belichtungszeit adaptiert; und
c. die Fokusebene des Objektivs 12 wird neu eingestellt. Wird eine Schwingung um eine Ruhelage senkrecht zum Bildsensor 10 (entlang der optischen Achse) erkannt, so wird die Fokusebene entsprechend eines für den nächsten Belichtungszeitraum vorhergesagter Position neu eingestellt und/oder die Schärfentiefe wird durch teilweises Schließen der Blende so eingestellt, dass ein hinreichend großer/der gesamte vertikale Schwingungsbereich hinreichend scharf abgebildet wird.

Zur Sensor-Bewegungserkennung kommen in Frage:
a. ein ein- bis sechsachsigen Beschleunigungssensor, der lineare Beschleunigungen entlang drei Achsen misst und sich auf der Bildaufnahmeeinheit oder dem Objekt befindet;
b. eine inertiale Messeinheit, welche lineare Beschleunigungen, z.B. entlang drei Achsen sowie Rotationen um drei Achsen misst ("Gyroskop") und ggf. noch das Magnetfeld misst (9DOF-absolute orientation sensors) und sich auf der Bildaufnahmeeinheit oder dem Objekt befindet;
c. eine Umfeldkamera mit weitem Blickwinkel, die ebenfalls auf der Bildaufnahmeeinheit sitzt, allerdings ein größeres oder anderes Bildfeld betrachtet (ggf. auch eine andere Richtung);
d. eine weitere Kamera oder allgemeiner ein externes Tracking-System, z.B. ein Laser-Tracking-System, welches nicht auf der Bildaufnahmeeinheit sitzt und die Bewegung der Bildaufnahmeeinheit von außen ermittelt. Hierfür können bei Bedarf auf der Bildaufnahmeeinheit zusätzlich Elemente wie Marker oder Retroreflektoren platziert werden;
e. eine Projektion von Markern/Muster ausgehend von der Bildaufnahmeeinheit und die Ermittlung der relativen Position der Marker/Muster zum Bildinhalt. Dabei können die Marker und der restliche Bildinhalt entweder im gleichen oder in einem bewusst trennbaren Spektralbereich (z.B. IR) liegen;
f. eine Projektion von Markern/Muster ausgehend von einem externen statischen Objekt;
g. ein Tracking von markanten Objektmerkmalen mit einem separatem Trackingsystem, wie beispielsweise ein Pupillentracking bei Operationsmikroskopen in der Ophthalmologie, und
h. ein zweiter Bildsensor, wie er ggf. in der Bildaufnahmeeinheit von Stereomikroskopsystemen vorhanden ist.

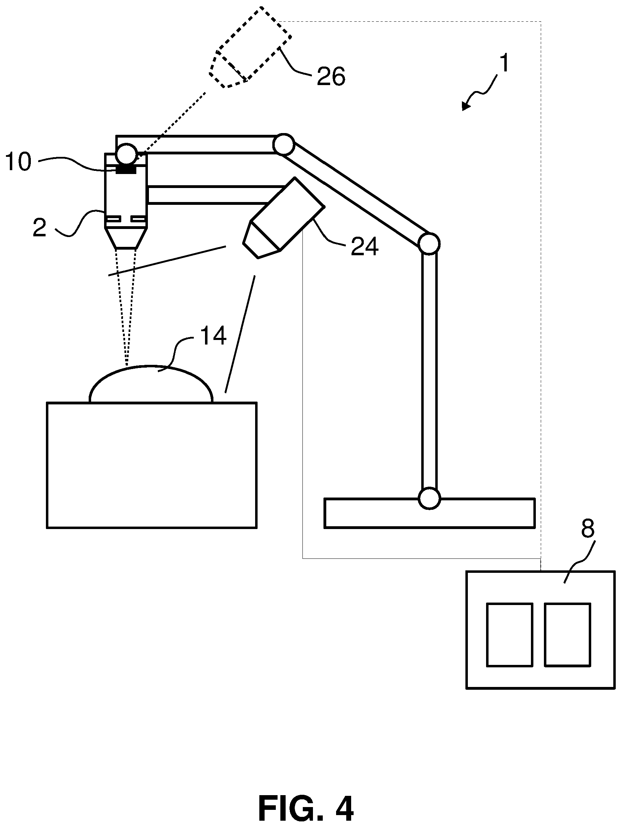

Fig. 4 zeigt exemplarisch die Ausführungsform mit Umfeldkamera 24 bzw. (gestrichelt) mit Tracking-System 26, welches die Bewegung des Mikroskopkopfs 2 und damit des Bildsensors 10 erfasst.

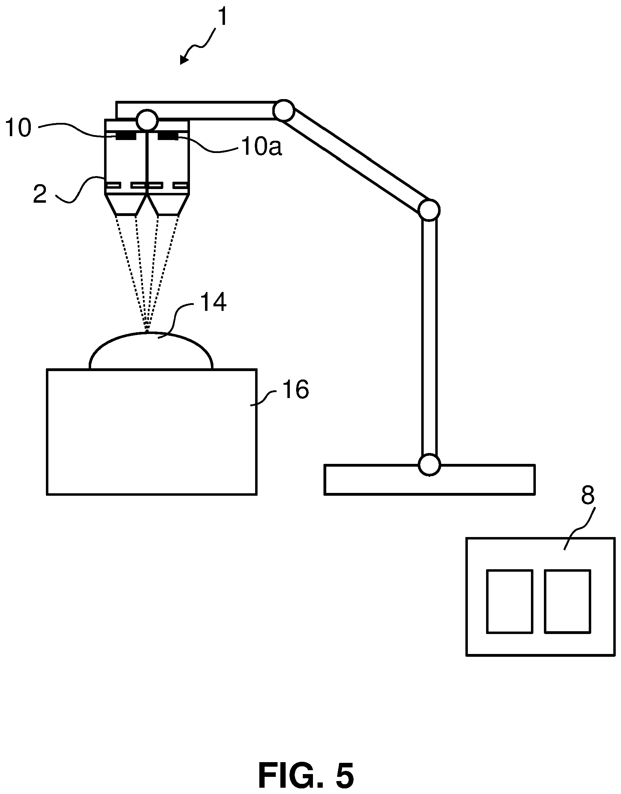

Fig. 5 zeigt exemplarisch die Ausgestaltung des Mikroskops 1 als Stereo-Mikroskop, so dass ein zweiter Bildsensor 10a vorgesehen ist.

Das Verfahren beschränkt sich nicht auf die Anwendung an Operationsmikroskopen, sondern kann allgemein auch auf andere Bereiche angewendet werden, bei denen eine Bildaufnahmeeinheit an einem sich bewegenden oder schwingenden Gegenstand montiert ist und ein sich ggf. bewegendes Objekt betrachtet wird.

## Patentansprüche

1. Verfahren zur Video-Stabilisierung bei einem medizinischen Visualisierungsystem (1), insbesondere ein OP-Mikroskop, umfassend einen Bildsensor (10), wobei das Verfahren folgende Schritte aufweist:
a) Bereitstellen des medizinischen Visualisierungsystems (1) und einer Bewegungserfassungseinrichtung (18), die eine Bewegung des Bildsensors (10) erfasst und entsprechende Sensor-Bewegungsdaten erzeugt,
b) Erfassen eines Objektfeldes (14) und Erzeugen von Video-Daten des Objektfeldes (14) mittels des Bildsensors (10) und Erzeugen von Bild-Bewegungsdaten, die Bewegungsveränderungen des Objektfeldes (14) wiedergeben, durch Auswerten der Video-Daten, und
c) Korrektur der Video-Daten umfassend
c1) Berechnen von Verschiebungsvektordaten, wobei die Sensor-Bewegungsdaten und die Bild-Bewegungsdaten verwendet und die Bild-Bewegungsdaten anhand der Sensor-Bewegungsdaten gewichtet und/oder gefiltert werden, so dass Verschiebungsvektordaten erhalten werden, die nur oder überwiegend eine Bewegung des Bildsensors (10), aber nicht oder nur untergeordnet eine Bewegung innerhalb des Objektfeldes wiedergeben, wobei anhand der Sensor-Bewegungsdaten ein Bewegungsvektor-Bereich definiert wird und nur Bild-Bewegungsdaten, die innerhalb dieses Bereichs liegen, für die Berechnung der Verschiebungsvektordaten verwendet werden, und
c2) Korrigieren der Video-Daten mittels der Verschiebungsvektordaten.

2. Verfahren nach Anspruch 1, wobei die errechneten Verschiebungsvektordaten in Form einer Matrix von Verschiebungsvektoren angeordnet werden und in Schritt c2) unter Verwendung der Matrix eine Bildverzerrung korrigiert wird.

3. Verfahren nach Anspruch 2, wobei ein Mittelwert der Verschiebungsvektoren in der Matrix berechnet und eine laterale Verschiebung korrigiert wird, wobei der Mittelwert verwendet wird.

4. Verfahren nach einem der obigen Ansprüche, wobei der Bildsensor im medizinischen Visualisierungsystem (1) an einem Stativ (4) oder Arm befestigt ist und in Schritt d1) die Verschiebungsvektordaten unter Verwendung eines Schwingungsmodell des Stativs (4) oder Arms berechnet werden.

5. Verfahren nach einem der obigen Ansprüche, wobei die Verschiebungsvektordaten daraufhin ausgewertet werden, ob eine axiale Schwingung vorliegt, die nur in einer Ebene senkrecht zum vom Bildsensor (10) erfassten Bildfeld verläuft, und wobei das medizinische Visualisierungsystem (1) eine Zoomoptik (12) aufweist und das Objekt (14) mittels zusätzlicher elektronischer Bildvergrößerung mit einer vorgegebenen Gesamtvergrößerung angezeigt wird und ein Anteil, den eine von der Zoomoptik (12) bewirkte Vergrößerung an der Gesamtvergrößerung hat, vergrößert oder maximiert wird, wenn die axiale Schwingung erkannt wurde.

6. Verfahren nach einem der obigen Ansprüche, wobei die Verschiebungsvektordaten daraufhin ausgewertet werden, ob eine laterale Schwingung vorliegt, die parallel zum vom Bildsensor (10) erfassten Bildfeld verläuft, und wobei
- das medizinische Visualisierungsystem (1) eine Zoomoptik (12) aufweist und das Objekt (14) mittels zusätzlicher elektronischer Bildvergrößerung mit einer vorgegebenen Gesamtvergrößerung angezeigt wird und ein Anteil, den die elektronische Bildvergrößerung an der Gesamtvergrößerung hat, vergrößert wird, wenn die laterale Schwingung erkannt wurde, und/oder
- das medizinische Visualisierungsystem (1) eine dem Bildsensor (10) vorgelagerte Pupillenblende (20) aufweist und diese unter Anpassung einer elektronischen Bildsensorsignalverstärkung vergrößert oder in der Öffnung maximiert wird, wenn die laterale Schwingung erkannt wurde.

7. Verfahren nach einem der obigen Ansprüche, wobei die Verschiebungsvektordaten daraufhin ausgewertet werden, ob eine axiale Schwingung vorliegt, die nur in einer Ebene senkrecht zum vom Bildsensor (10) erfassten Bildfeld verläuft, und wobei
- das medizinische Visualisierungsystem (1) eine dem Bildsensor (10) vorgelagerte Pupillenblende (20) aufweist und diese unter Anpassung einer elektronischen Bildsensorsignalverstärkung verkleinert oder in der Öffnung minimiert wird, wenn die axiale Schwingung erkannt wurde, und/oder
- das medizinische Visualisierungsystem (1) eine Fokussiereinrichtung (12) aufweist und diese zur Änderung der Fokuslage angesteuert wird, wenn die axiale Schwingung erkannt wurde.

8. Medizinisches Visualisierungsystem, insbesondere ein OP-Mikroskopsystem, umfassend
- einen Bildsensor (10) zum Erzeugen von Video-Daten für ein Objekt (14) und eine Bewegungserfassungseinrichtung (18), die konfiguriert ist, eine Bewegung des Bildsensors (10) zu erfassen und entsprechende Sensor-Bewegungsdaten zu erzeugen,
- eine Steuereinrichtung (8), die einen Prozessor (8.1) und einen Speicher (8.2) umfasst, mit dem Bildsensor (10) und der Bewegungserfassungseinrichtung (18) über eine Datenverbindung verbunden ist,
- eine Anzeige (22) zur Anzeige der Video-Daten,
- wobei die Steuereinrichtung (8) konfiguriert ist,
-- ein Objektfeld (14) zu erfassen und Bild-Bewegungsdaten, die Bewegungsveränderungen des Objektfeldes (14) wiedergeben, durch Auswerten der Video-Daten zu erzeugen,
-- Verschiebungsvektordaten zu errechnen und dabei die Sensor-Bewegungsdaten und der Bild-Bewegungsdaten zu verwenden und die Bewegungsveränderungen des Objektfeldes wiedergebenden Bild-Bewegungsdaten anhand der Sensor-Bewegungsdaten zu gewichten und/oder filtern, so dass die Verschiebungsvektordaten nur oder überwiegend eine Bewegung des Bildsensors (10), aber nicht oder nur untergeordnet eine Bewegung innerhalb des Objektfeldes wiedergeben, wobei die Steuereinrichtung (8) konfiguriert ist. anhand der Sensor-Bewegungsdaten einen Bewegungsvektor-Bereich zu definieren und nur Bild-Bewegungsdaten, die innerhalb dieses Bereichs liegen, für die Berechnung der Verschiebungsvektordaten zu verwenden, und
-- die Video-Daten mittels der Verschiebungsvektordaten zu korrigieren und an die Anzeige (22) zu leiten.

9. Medizinisches Visualisierungsystem nach Anspruch 8, wobei der Bildsensor (10) an einem Stativ (4) oder Arm befestigt ist und die Steuereinrichtung (8) weiter konfiguriert ist, ein Schwingungsmodell des Stativs (4) oder Arms verwendet wird, um die Verschiebungsvektordaten zu berechnen.

10. Medizinisches Visualisierungsystem nach Anspruch 8 oder 9, wobei die Steuereinrichtung (8) konfiguriert ist, die errechneten Verschiebungsvektordaten in Form einer Matrix von Verschiebungsvektoren anzuordnen, und die Steuereinrichtung (8) weiter konfiguriert ist, unter Verwendung der Matrix eine Bildverzerrung zu korrigieren.

11. Medizinisches Visualisierungsystem nach Anspruch 10, wobei die Steuereinrichtung (8) konfiguriert ist, einen Mittelwert der Verschiebungsvektoren in der Matrix zu berechnen und unter Verwendung der Matrix zur Korrektur einer lateralen Verschiebung zu nutzen.

12. Medizinisches Visualisierungsystem nach einem der Ansprüche 8 bis 11, wobei die Steuereinrichtung konfiguriert ist, die Verschiebungsvektordaten daraufhin auszuwerten, ob eine axiale Schwingung vorliegt, die nur in einer Ebene senkrecht zum vom Bildsensor (10) erfassten Bildfeld verläuft, und wobei
- das medizinische Visualisierungsystem (1) eine von der Steuereinrichtung (8) angesteuerte Zoomoptik (12) und eine Anzeige (22) aufweist und das Objekt (14) auf der Anzeige (22) mittels zusätzlicher elektronischer Bildvergrößerung mit einer vorgegebenen Gesamtvergrößerung anzeigt und die Steuereinrichtung (8) weiter konfiguriert ist, einen Anteil, den eine von der Zoomoptik (12) bewirkte Vergrößerung an der Gesamtvergrößerung hat, zu vergrößern oder zu maximieren, wenn die axiale Schwingung erkannt wurde, und/oder
- das medizinische Visualisierungsystem (1) eine dem Bildsensor (10) vorgelagerte, von der Steuereinrichtung (8) angesteuerte Pupillenblende (20) aufweist und die Steuereinrichtung (8) weiter konfiguriert ist, die Pupillenblende (20) unter Anpassung einer elektronischen Bildsensorsignalverstärkung zu verkleinern oder in der Öffnung zu minimieren, wenn die axiale Schwingung erkannt wurde, und/oder
- das medizinische Visualisierungsystem (1) eine von der Steuereinrichtung (8) angesteuerte Fokussiereinrichtung (12) aufweist und die Steuereinrichtung (8) weiter konfiguriert ist, die Fokussiereinrichtung (12) zur Änderung der Fokuslage anzusteuern, wenn die axiale Schwingung erkannt wurde.

13. Medizinisches Visualisierungsystem nach einem der Ansprüche 8 bis 12, wobei die Steuereinrichtung konfiguriert ist, die Verschiebungsvektordaten daraufhin auszuwerten, ob eine parallele Schwingung vorliegt, die in einer Ebene parallel zum vom Bildsensor (10) erfassten Bildfeld verläuft, und wobei
- das medizinische Visualisierungsystem (1) eine von der Steuereinrichtung (8) angesteuerte Zoomoptik (12) aufweist und das Objekt (14) mittels zusätzlicher elektronischer Bildvergrößerung auf der Anzeige (22) mit einer vorgegebenen Gesamtvergrößerung anzeigt und die Steuereinrichtung (8) weiter konfiguriert ist, einen Anteil, den die elektronische Bildvergrößerung an der Gesamtvergrößerung hat, zu vergrößern, wenn die parallele Schwingung erkannt wurde, und/oder
- das medizinische Visualisierungsystem (1) eine dem Bildsensor (10) vorgelagerte, von der Steuereinrichtung (8) angesteuerte Pupillenblende (20) aufweist und die Steuereinrichtung (8) weiter konfiguriert ist, die Pupillenblende (20) unter Anpassung einer elektronischen Bildsensorsignalverstärkung zu vergrößern oder in der Öffnung zu maximieren, wenn die parallele Schwingung erkannt wurde.

14. Medizinisches Visualisierungsystem nach einem der Ansprüche 8 bis 13, **dadurch gekennzeichnet, dass** die Bewegungserfassungseinrichtung (18) mindestens eine der folgenden Einrichtungen aufweist: einen ein- bis sechsachsigen Beschleunigungssensor in fester Ortslage zum Bildsensor (10), ein ein- bis sechsachsiges Intertialmesssystem in fester Ortslage zum Bildsensor (10), eine Umfeldkamera (24) in fester Ortslage zum Bildsensor (10), ein den Bildsensor (10) direkt oder mittelbar überwachendes Trackingsystem (26), einen ein Muster auf das vom Bildsensor (10) erfasste Objekt (14) projizierenden Musterprojektor in fester Ortslage zum Bildsensor (10), ein das Objekt (14) direkt oder mittelbar überwachendes Trackingsystem (26), einen Pupillentracker und einen zweiten Bildsensor (100a), der unter einem Stereowinkel auf das Objekt (14) blickt.

## Claims

1. Method for video stabilization in a medical visualization system (1), in particular a surgical microscope, comprising an image sensor (10), wherein the method includes the following steps:
a) providing the medical visualization system (1) and a movement capture device (18) that captures a movement of the image sensor (10) and generates corresponding sensor movement data,
b) capturing an object field (14) and generating video data of the object field (14) by means of the image sensor (10) and generating image movement data which reproduce movement changes in the object field (14), by evaluating the video data, and
c) correcting the video data, comprising
c1) calculating displacement vector data, wherein the sensor movement data and the image movement data are used and the image movement data are weighted and/or filtered on the basis of the sensor movement data, so that displacement vector data are obtained which only or predominantly reproduce a movement of the image sensor (10), but do not, or only to a minor degree, reproduce a movement within the object field, wherein a movement vector range is defined on the basis of the sensor movement data and only image movement data which lie within this range are used for the calculation of the displacement vector data, and
c2) correcting the video data by means of the displacement vector data.

2. Method according to Claim 1, wherein the calculated displacement vector data are arranged in the form of a matrix of displacement vectors and in step c2) an image distortion is corrected using the matrix.

3. Method according to Claim 2, wherein a mean value of the displacement vectors in the matrix is calculated and a lateral displacement is corrected, wherein the mean value is used.

4. Method according to any of the above claims, wherein the image sensor in the medical visualization system (1) is attached to a stand (4) or arm and in step d1) the displacement vector data are calculated using a vibration model of the stand (4) or arm.

5. Method according to any of the above claims, wherein the displacement vector data are evaluated to determine whether an axial vibration is present, which runs only in one plane perpendicular to the image field captured by the image sensor (10), and wherein the medical visualization system (1) has a zoom optical unit (12) and the object (14) is displayed with a specified total magnification by means of additional electronic image magnification, and a proportion of the total magnification which a magnification that is due to the zoom optical unit (12) has is enlarged or maximized when the axial vibration has been detected.

6. Method according to any of the above claims, wherein the displacement vector data are evaluated to determine whether a lateral vibration is present, which runs parallel to the image field captured by the image sensor (10), and wherein
- the medical visualization system (1) has a zoom optical unit (12) and the object (14) is displayed with a specified total magnification by means of additional electronic image magnification and a proportion of the total magnification which the electronic image magnification has is enlarged when the lateral vibration has been detected, and/or
- the medical visualization system (1) has a pupil stop (20) upstream of the image sensor (10) and this pupil stop is enlarged by adapting an electronic image sensor signal gain or maximized in terms of the opening when the lateral vibration has been detected.

7. Method according to any of the above claims, wherein the displacement vector data are evaluated to determine whether an axial vibration is present, which runs only in one plane perpendicular to the image field captured by the image sensor (10), and wherein
- the medical visualization system (1) has a pupil stop (20) upstream of the image sensor (10) and this pupil stop is decreased by adapting an electronic image sensor signal gain or minimized in terms of the opening when the axial vibration has been detected, and/or
- the medical visualization system (1) has a focusing device (12) and the latter is controlled to change the focal position when the axial vibration has been detected.

8. Medical visualization system, in particular a surgical microscope system, comprising
- an image sensor (10) for generating video data for an object (14) and a movement capture device (18) configured to capture a movement of the image sensor (10) and to generate corresponding sensor movement data,
- a control device (8) comprising a processor (8.1) and a memory (8.2) which is connected to the image sensor (10) and the movement capture device (18) via a data link,
- a display (22) for displaying the video data,
- wherein the control device (8) is configured
-- to capture an object field (14) and to generate image movement data which reproduce movement changes in the object field (14), by evaluating the video data,
-- to calculate displacement vector data and in the process to use the sensor movement data and the image movement data and to weight and/or filter the image movement data reproducing movement changes in the object field on the basis of the sensor movement data, so that the displacement vector data only or predominantly reproduce a movement of the image sensor (10), but do not, or only to a minor degree, reproduce a movement within the object field, wherein the control device (8) is configured to define a movement vector range on the basis of the sensor movement data and to use only image movement data which lie within this range for the calculation of the displacement vector data, and
-- to correct the video data by means of the displacement vector data and to transmit them to the display (22).

9. Medical visualization system according to Claim 8, wherein the image sensor (10) is attached to a stand (4) or arm and the control device (8) is further configured to use a vibration model of the stand (4) or arm to calculate the displacement vector data.

10. Medical visualization system according to Claim 8 or 9, wherein the control device (8) is configured to arrange the calculated displacement vector data in the form of a matrix of displacement vectors, and the control device (8) is further configured to correct an image distortion using the matrix.

11. Medical visualization system according to Claim 10, wherein the control device (8) is configured to calculate a mean value of the displacement vectors in the matrix and to use it for correcting a lateral displacement using the matrix.

12. Medical visualization system according to any of Claims 8 to 11, wherein the control device is configured to evaluate the displacement vector data to determine whether an axial vibration is present, which runs only in one plane perpendicular to the image field captured by the image sensor (10), and wherein
- the medical visualization system (1) has a zoom optical unit (12), controlled by the control device (8), and a display (22), and displays the object (14) on the display (22) with a specified total magnification by means of additional electronic image magnification, and the control device (8) is further configured to enlarge or maximize a proportion of the total magnification which a magnification that is due to the zoom optical unit (12) has when the axial vibration has been detected, and/or
- the medical visualization system (1) has a pupil stop (20) which is arranged upstream of the image sensor (10) and is controlled by the control device (8), and the control device (8) is further configured to decrease the pupil stop (20) by adapting an electronic image sensor signal gain or minimize it in terms of the opening when the axial vibration has been detected, and/or
- the medical visualization system (1) has a focusing device (12) controlled by the control device (8), and the control device (8) is further configured to control the focusing device (12) for changing the focal position when the axial vibration has been detected.

13. Medical visualization system according to any of Claims 8 to 12, wherein the control device is configured to evaluate the displacement vector data to determine whether a parallel vibration is present, which runs in a plane parallel to the image field captured by the image sensor (10), and wherein
- the medical visualization system (1) has a zoom optical unit (12), controlled by the control device (8), and displays the object (14) on the display (22) with a specified total magnification by means of additional electronic image magnification, and the control device (8) is further configured to enlarge a proportion of the total magnification which the electronic image magnification has when the parallel vibration has been detected, and/or
- the medical visualization system (1) has a pupil stop (20) which is arranged upstream of the image sensor (10) and is controlled by the control device (8), and the control device (8) is further configured to enlarge the pupil stop (20) by adapting an electronic image sensor signal gain or maximize it in terms of the opening when the parallel vibration has been detected.

14. Medical visualization system according to any of Claims 8 to 13, **characterized in that** the movement capture device (18) has at least one of the following devices: a single-axis to six-axis acceleration sensor in a fixed location relative to the image sensor (10), a single-axis to six-axis inertial measurement system in a fixed location relative to the image sensor (10), a vicinity camera (24) in a fixed location relative to the image sensor (10), a tracking system (26) directly or indirectly monitoring the image sensor (10), a pattern projector, which projects a pattern onto the object (14) captured by the image sensor (10), in a fixed location relative to the image sensor (10), a tracking system (26) directly or indirectly monitoring the object (14), a pupil tracker and a second image sensor (100a) that looks at the object (14) at a stereo angle.

## Revendications

1. Procédé de stabilisation vidéo dans un système de visualisation médical (1), en particulier un microscope opératoire, comprenant un capteur d'image (10), le procédé comprenant les étapes suivantes :
a) la mise à disposition du système de visualisation médical (1) et d'un dispositif de détection de mouvement (18), qui détecte un mouvement du capteur d'image (10) et génère des données de mouvement de capteur correspondantes,
b) la détection d'un champ d'objet (14) et la génération de données vidéo du champ d'objet (14) au moyen du capteur d'image (10) et la génération de données de mouvement d'image, qui représentent des modifications de mouvement du champ d'objet (14), par évaluation des données vidéo, et
c) la correction des données vidéo, comprenant
c1) le calcul de données de vecteurs de décalage, les données de mouvement de capteur et les données de mouvement d'image étant utilisées et les données de mouvement d'image étant pondérées et/ou filtrées sur la base des données de mouvement de capteur de manière à obtenir des données de vecteurs de décalage qui représentent uniquement ou principalement un mouvement du capteur d'image (10), mais pas ou seulement de manière subordonnée un mouvement à l'intérieur du champ d'objet, une plage de vecteurs de mouvement étant définie sur la base des données de mouvement de capteur et seules les données de mouvement d'image qui se trouvent à l'intérieur de cette plage étant utilisées pour le calcul des données de vecteurs de décalage, et
c2) la correction des données vidéo au moyen des données de vecteurs de décalage.

2. Procédé selon la revendication 1, dans lequel les données de vecteurs de décalage calculées sont agencées sous la forme d'une matrice de vecteurs de décalage et, à l'étape c2), une distorsion d'image est corrigée à l'aide de la matrice.

3. Procédé selon la revendication 2, dans lequel une moyenne des vecteurs de décalage dans la matrice est calculée et un décalage latéral est corrigé à l'aide de la moyenne.

4. Procédé selon l'une des revendications précédentes, dans lequel le capteur d'image est fixé à un statif (4) ou à un bras dans le système de visualisation médical (1) et, à l'étape d1), les données de vecteurs de décalage sont calculées à l'aide d'un modèle de vibration du statif (4) ou du bras.

5. Procédé selon l'une des revendications précédentes, dans lequel les données de vecteurs de décalage sont évaluées afin de déterminer s'il existe une oscillation axiale s'étendant uniquement dans un plan perpendiculaire au champ d'image détecté par le capteur d'image (10), et dans lequel le système de visualisation médical (1) comporte une optique de zoom (12) et l'objet (14) est affiché au moyen d'un agrandissement d'image électronique supplémentaire avec un grossissement total prédéfini, et une part du grossissement total représentée par l'agrandissement produit par l'optique de zoom (12) est augmentée ou maximisée lorsque l'oscillation axiale a été identifiée.

6. Procédé selon l'une des revendications précédentes, dans lequel les données de vecteurs de décalage sont évaluées afin de déterminer s'il existe une oscillation latérale s'étendant parallèlement au champ d'image détecté par le capteur d'image (10), et dans lequel
- le système de visualisation médical (1) comporte une optique de zoom (12) et l'objet (14) est affiché au moyen d'un agrandissement d'image électronique supplémentaire avec un grossissement total prédéfini, et une part du grossissement total représentée par l'agrandissement d'image électronique est augmentée lorsque l'oscillation latérale a été identifiée, et/ou
- le système de visualisation médical (1) comporte un diaphragme de pupille (20) placé en amont du capteur d'image (10) et celui-ci est agrandi ou son ouverture est maximisée en adaptant une amplification de signal du capteur d'image électronique lorsque l'oscillation latérale a été identifiée.

7. Procédé selon l'une des revendications précédentes, dans lequel les données de vecteurs de décalage sont évaluées afin de déterminer s'il existe une oscillation axiale s'étendant uniquement dans un plan perpendiculaire au champ d'image détecté par le capteur d'image (10), et dans lequel
- le système de visualisation médical (1) comporte un diaphragme de pupille (20) placé en amont du capteur d'image (10) et celui-ci est réduit ou son ouverture est minimisée en adaptant une amplification de signal du capteur d'image électronique lorsque l'oscillation axiale a été identifiée, et/ou
- le système de visualisation médical (1) comporte un dispositif de focalisation (12) qui est commandé pour modifier la position focale lorsque l'oscillation axiale a été identifiée.

8. Système de visualisation médical, en particulier système de microscope opératoire, comprenant
- un capteur d'image (10) pour générer des données vidéo pour un objet (14) et un dispositif de détection de mouvement (18) configuré pour détecter un mouvement du capteur d'image (10) et pour générer des données de mouvement de capteur correspondantes,
- un dispositif de commande (8) comprenant un processeur (8.1) et une mémoire (8.2), relié au capteur d'image (10) et au dispositif de détection de mouvement (18) par une liaison de données,
- un dispositif d'affichage (22) pour afficher les données vidéo,
- dans lequel le dispositif de commande (8) est configuré pour
-- détecter un champ d'objet (14) et générer des données de mouvement d'image qui représentent des modifications de mouvement du champ d'objet (14), par évaluation des données vidéo,
-- calculer des données de vecteurs de décalage en utilisant pour cela les données de mouvement de capteur et les données de mouvement d'image, et pondérer et/ou filtrer les données de mouvement d'image représentant les modifications de mouvement du champ d'objet sur la base des données de mouvement de capteur, de sorte que les données de vecteurs de décalage représentent uniquement ou principalement un mouvement du capteur d'image (10), mais pas ou seulement de manière subordonnée un mouvement à l'intérieur du champ d'objet, le dispositif de commande (8) étant configuré pour définir une plage de vecteurs de mouvement sur la base des données de mouvement de capteur et utiliser uniquement les données de mouvement d'image situées à l'intérieur de cette plage pour le calcul des données de vecteurs de décalage, et
-- corriger les données vidéo au moyen des données de vecteurs de décalage et les transmettre au dispositif d'affichage (22).

9. Système de visualisation médical selon la revendication 8, dans lequel le capteur d'image (10) est fixé à un statif (4) ou à un bras et le dispositif de commande (8) est en outre configuré pour utiliser un modèle de vibration du statif (4) ou du bras pour calculer les données de vecteurs de décalage.

10. Système de visualisation médical selon la revendication 8 ou 9, dans lequel le dispositif de commande (8) est configuré pour agencer les données de vecteurs de décalage calculées sous la forme d'une matrice de vecteurs de décalage, et le dispositif de commande (8) est en outre configuré pour corriger une distorsion d'image à l'aide de la matrice.

11. Système de visualisation médical selon la revendication 10, dans lequel le dispositif de commande (8) est configuré pour calculer une valeur moyenne des vecteurs de décalage dans la matrice et pour l'utiliser afin de corriger un décalage latéral à l'aide de la matrice.

12. Système de visualisation médical selon l'une des revendications 8 à 11, dans lequel le dispositif de commande est configuré pour évaluer les données de vecteurs de décalage afin de déterminer s'il existe une oscillation axiale s'étendant uniquement dans un plan perpendiculaire au champ d'image détecté par le capteur d'image (10), et dans lequel
- le système de visualisation médical (1) comporte une optique de zoom (12) commandée par le dispositif de commande (8), et un dispositif d'affichage (22), et affiche l'objet (14) sur le dispositif d'affichage (22) au moyen d'un agrandissement d'image électronique supplémentaire avec un grossissement total prédéfini, et le dispositif de commande (8) est en outre configuré pour augmenter ou maximiser une part du grossissement total représentée par le grossissement produit par l'optique de zoom (12) lorsque l'oscillation axiale a été identifiée, et/ou
- le système de visualisation médical (1) comporte un diaphragme de pupille (20) placé en amont du capteur d'image (8) et commandé par le dispositif de commande (20), et le dispositif de commande (10) est en outre configuré pour réduire le diaphragme de pupille (8) ou pour minimiser son ouverture en adaptant une amplification de signal du capteur d'image électronique lorsque l'oscillation axiale a été identifiée, et/ou
- le système de visualisation médical (1) comporte un dispositif de focalisation (12) commandé par le dispositif de commande (8), et le dispositif de commande (8) est en outre configuré pour commander le dispositif de focalisation (12) afin de modifier la position focale lorsque l'oscillation axiale a été identifiée.

13. Système de visualisation médical selon l'une des revendications 8 à 12, dans lequel le dispositif de commande est configuré pour évaluer les données de vecteurs de décalage afin de déterminer s'il existe une oscillation parallèle s'étendant dans un plan parallèle au champ d'image détecté par le capteur d'image (10), et dans lequel
- le système de visualisation médical (1) comporte une optique de zoom (12) commandée par le dispositif de commande (8) et affiche l'objet (14) sur le dispositif d'affichage (22) au moyen d'un agrandissement d'image électronique supplémentaire avec un grossissement total prédéfini, et le dispositif de commande (8) est en outre configuré pour augmenter une part du grossissement total représentée par l'agrandissement d'image électronique lorsque l'oscillation parallèle a été identifiée, et/ou
- le système de visualisation médical (1) comporte un diaphragme de pupille (20) placé en amont du capteur d'image (10) et commandé par le dispositif de commande (8), et le dispositif de commande (8) est en outre configuré pour agrandir le diaphragme de pupille (20) ou maximiser son ouverture en adaptant une amplification de signal du capteur d'image électronique, lorsque l'oscillation parallèle a été identifiée.

14. Système de visualisation médical selon l'une des revendications 8 à 13, **caractérisé en ce que** le dispositif de détection de mouvement (18) comporte au moins l'un des dispositifs suivants : un capteur d'accélération à un à six axes dans une position spatiale fixe par rapport au capteur d'image (10), un système de mesure inertielle à un à six axes dans une position spatiale fixe par rapport au capteur d'image (10), une caméra d'environnement (24) située dans une position spatiale fixe par rapport au capteur d'image (10), un système de poursuite (26) surveillant directement ou indirectement le capteur d'image (10), un projecteur de motif projetant un motif sur l'objet (14) détecté par le capteur d'image (10) dans une position spatiale fixe par rapport au capteur d'image (10), un système de poursuite (26) surveillant directement ou indirectement l'objet (14), un dispositif de poursuite de pupille et un second capteur d'image (100a) regardant l'objet (14) sous un angle stéréoscopique.
